# EUROPÄISCHE PATENTSCHRIFT

(11) **EP 1 464 342 B1**
(45) Veröffentlichungstag und Bekanntmachung des Hinweises auf die Patenterteilung: **07.10.2009**
(21) Anmeldenummer: 04006661.5
(22) Anmeldetag: 19.03.2004
(51) Int. Cl.: A61L 2/00, A61L 2/04, A61L 2/10, A23L 3/005, A23L 3/28, A23L 3/22, C02F 1/32

(54) **Vorrichtung und Verfahren zur Sterilisation flüssiger Medien mittels UV-Bestrahlung und Kurzzeiterhitzung**
Apparatus and process for sterilising liquids using ultraviolet radiation and short-time heating
Dispositif et procédé de stérilisation des liquides par rayonnement ultraviolet et traitement thermique de courte durée

(30) Priorität: 21.03.2003 DE 10312765
(43) Veröffentlichungstag der Anmeldung: 06.10.2004
(73) Patentinhaber: Bayer Technology Services GmbH, 51368 Leverkusen (DE)
(72) Erfinder: Kaiser, Klaus, Dr., 51105 Köln (DE); Kauling, Jörg, 51069 Köln (DE); Henzler, Hans-Jürgen, Dr., 42655 Solingen (DE); Schmidt, Sebastian, Dr., 42781 Haan (DE); Schmitt, Franz, 51465 Bergisch Gladbach (DE); Beckers, Erhard, 51399 Burscheid (DE)

(56) Entgegenhaltungen:
- WO-A-00/56160
- WO-A-01/37675
- WO-A-01/91811
- WO-A-02/38502
- WO-A-99/34910
- DE-A- 1 951 633
- US-A- 1 141 056
- US-A- 3 934 042
- US-A- 4 534 282

## Beschreibung

Die Erfindung betrifft eine Technik zur sicheren und produktschonenden UV-Bestrahlung und Hitze-Sterilisierung flüssiger Medien und insbesondere Mikroorganismen und/oder Viren enthaltender Flüssigkeiten (Nahrungsmittel, Milch- oder Fruchtsaftprodukte, chemische oder pharmazeutische Produkten, Virus-Vakzine, gentechnisch erzeugte Wirkstoffe oder Proteine, Wirkstoffe oder Proteine aus transgenen Tieren oder Pflanzen und Blutplasma oder aus Blutplasma gewonnene Produkte). Ein gemeinsames Merkmal der Bestrahlung mit UV-Licht und der Behandlung mit Hitze ist eine die Inaktivierungsreaktion begleitende unerwünschte Produktschädigung, deren Ausmaß durch geeignete reaktionstechnische und konstruktive Maßnahmen minimiert werden muss.

Das Verfahren zur Sterilisation flüssiger Medien basiert auf der zeitnahen Anwendung der beiden Verfahrensschritte einer Hitze- und UVC(UV Strahlung der C-Kategorie) - Behandlung (UV Strahlung der C-Kategorie), die in der kombinierten Anwendung über synergetische Effekte eine besonders produktschonende Abtötung von Mikroorganismen und Viren erlauben. Als Reaktoren werden wendelförmige Strömungskanäle mit einem produktseitig eingeengten Verweilzeitspektrum verwendet. Die Produktkanäle werden durch Aufziehen eines spiralförmig gewellten Schlauchkörpers auf einen für Wärme oder UV-Strahlen durchlässigen zylindrischen Rohrkörper erzeugt. Zur Vermeidung einer schwer zu validierbaren Reinigung sind die Wendelreaktoren so konstruiert, dass sie nach der Produktbehandlung durch neue, exakt qualifizierte und sterilisierte Reaktoren ersetzt werden können.

Die Sterilisation flüssiger Medien ist eine wesentliche Ausgangsvoraussetzung für den Einsatz biotechnologischer Produktionsverfahren in der Lebensmittel- und Pharmaindustrie. Zielsetzung ist die zuverlässige und weitgehende Abtötung von Mikroorganismen und/oder Viren bei gleichzeitig weitgehender Erhaltung der empfindlichen Wertstoffe. Hauptanwendungsgebiete der Sterilisationsverfahren sind die sterile Durchführung von Fermentationsprozessen, die Haltbarkeitsverlängerung durch sterile bzw. keimarme Lebensmittelabfüllung sowie die pharmazeutische Verwendbarkeit biologischer Wirkstoffe humanen oder tierischen Ursprungs z.B. aus Organen oder Blutplasma. Für die Anwendung biologischer Wirkstoffe wird von der FDA ein validierungspflichtiger Sterilisationsprozess mit mehreren, auf unterschiedlichen Wirkprinzipien basierenden Virusinaktivierungsschritten gefordert. Die Validierbarkeit der Sterilisationsverfahren setzt voraus, dass sich die verwendeten Reaktoren und Anlagen in einem exakt spezifizierbaren Zustand befinden. Eine Verschleppung von Kontaminationen über Prozesschargen hinweg muss dabei ausgeschlossen werden.

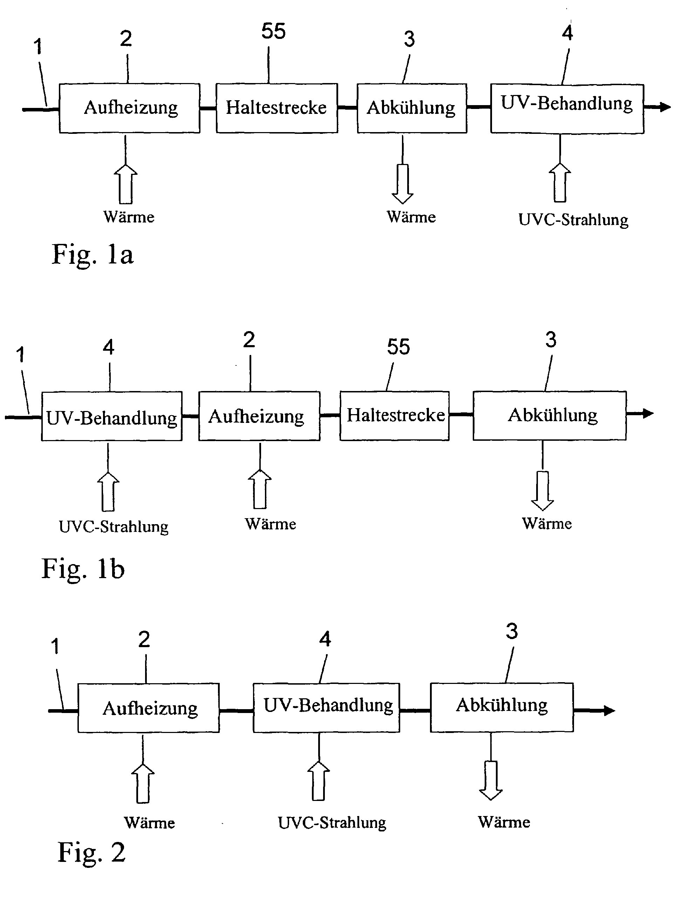

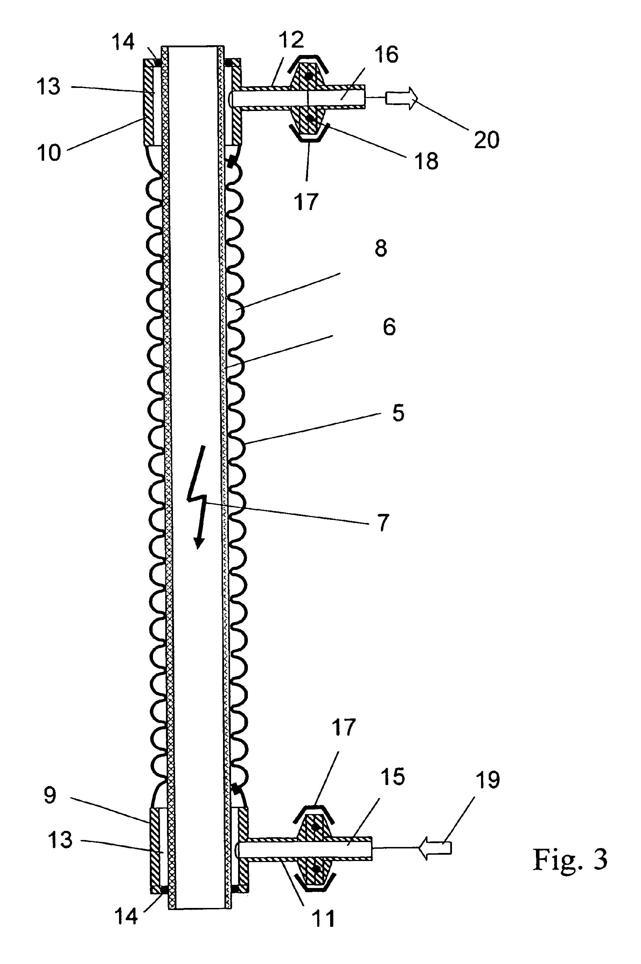

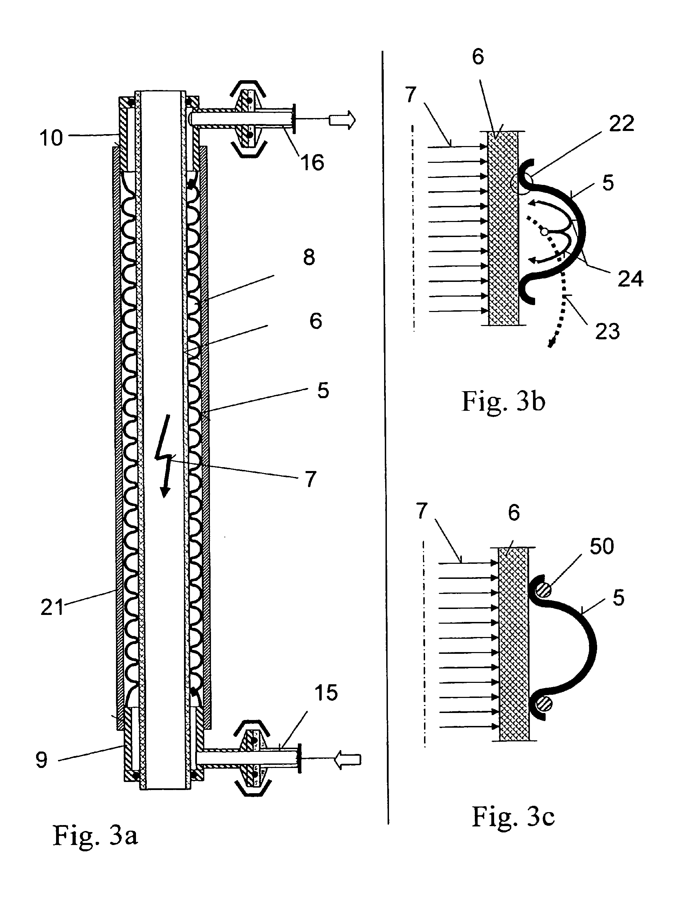

Ein wichtiges Kriterium zur Produktschonung ist die Verkürzung der Produktexposition in der Reaktionszone. Da die notwendige mittlere Behandlungsdauer durch die am schnellsten die Reaktionszone passierenden Teilchen festgelegt wird, besteht zur Reduzierung der Behandlungsdauer die Forderung nach einer möglichst einheitlichen Verweilzeitverteilung innerhalb des Produktstroms. In der Literatur [EP A1 1 339 643, EP A1 1 337 280, VDI Wärmeatlas] wird das besonders günstige Verweilzeitverhalten in wendelförmigen Strömungskanälen beschrieben, das durch senkrecht zur Strömungsrichtung (Fig. 3b, (23)) wirkenden Sekundärströmungen (sog. Deanwirbel) verursacht wird (24). Wie Untersuchungen zur Inaktivierung von einem Modellvirus gezeigt haben, ist es erstmals gelungen, eine gleichmäßige, exakt kontrollierbare Behandlung der Produktlösungen zu realisieren. Jedes Flüssigkeitselement wird bei der Durchströmung in die unmittelbare Nähe der Behandlungsquelle geführt und damit der inaktivierenden UV-Strahlung bzw. Wärme ausgesetzt.

Neben der verbesserten Durchströmung ist gefunden worden, dass die Kombination der unter geeigneten Bedingungen (Temperatur und UV-Bestrahlungsdosis) durchgeführten kurzzeitigen Wärme- und UV-Behandlung besonders vorteilhaft ist. Durch die kurzzeitig aufeinanderfolgende (UV-Behandlung des Produktstroms nach Aufheizung und Abkühlung oder UV-Behandlung des Produktstroms vor Aufheizung und Abkühlung) oder sich zeitlich überschneidende Abfolge beider Verfahrensschritte (Aufheizung des Produktstroms, UV-Behandlung und Abkühlung) wird ein zusätzliches synergetisches Inaktivierungspotential induziert. Dies führt bei gleichem Inaktivierungserfolg überraschenderweise zu einer Reduzierung der erforderlichen Energiebelastung und somit zu einer Verringerung der Produktschädigung im Gesamtprozess. Die Anwendung der thermischen Sterilisationstechnik erfordert mindestens zwei Reaktoren, einen für die Aufheizung und einen für die anschließende Abkühlung. Zur Konstanthaltung der Produkttemperatur kann optional eine gut wärmeisolierte Rohrleitung als Haltestrecke zwischen Heiz- und Kühlreaktor geschaltet werden. Bei einer innerhalb der thermischen Behandlung durchgeführten UV-Behandlung dient der UV-Reaktor auch als Haltemodul.

Gegenstand der Erfindung ist eine Vorrichtung geeignet zur Durchführung des erfindungsgemäßen Verfahrens bestehend wenigstens aus einem Wärmebehandlungsreaktor, gegebenenfalls einem Temperaturhaltestrecke, einem UV-Bestrahlungsreaktor und einem Kühlreaktor, dadurch gekennzeichnet, dass der vom Reaktionsmedium (Produkt) durchströmte Sterilisationsund/oder Inaktivierungsraum mindestens des Bestrahlungsreaktors und des Wärmebehandlungsreaktors durch einen verformbaren wendelförmigen profilierten Hohlzylinder gebildet wird, der auf einen für eingesetzte Sterilisations- oder Inaktivierungsenergie durchlässigen, starren, geraden, zylindrischen Stützkörper kraftschlüssig aufgezogen ist.

Als verformbarer wendelförmiger Hohlzylinder wird bevorzugt ein Wellschlauch aus Kunststoff verwendet, der zur Produktzu- bzw. -abfuhr an beiden Enden mit Verteilerköpfen verbunden ist.

Besonders bevorzugt ist eine Vorrichtung, dadurch gekennzeichnet, dass die Verteilerköpfe eine totraumfreie tangentiale oder vorzugsweise radiale Produktzu- bzw. -ableitung im Bereich des Ringspaltes zwischen Verteilerkopf und Stützrohr aufweisen.

Bevorzugt ist auch eine Vorrichtung, dadurch gekennzeichnet, dass die Verteilerköpfe (9, 10) durch eine nachträgliche thermische Verformung des Wellschlauches aus den Schlauchenden herausgearbeitet wird oder bevorzugt aus einem im Spritzgussverfahren und/oder spannend hergestellten bzw. bearbeiteten Kunststoffmaterial hergestellt sind und mittels einer von außen angepressten O-Ring-Verbindung (32, 5, 33) mit den zylindrisch aufgeweiteten Schlauchenden kraftschlüssig verbunden sind.

Zur Verbindung der Ausdehnung weist der Wellschlauch in einer bevorzugten Ausführung einen Außenmantel oder eine Armierung auf.

Besonders bevorzugt ist der Außenmantel durch einen schrumpfbaren Kunststoffschlauch, ein über den Spiralschlauch geschobenes Rohr oder vorzugsweise eine zweigeteilte, zylindrische Schale gebildet, wobei die Armierung aus einer Stahl- oder Kunststoffwendel besteht.

Der UV-Reaktor weist bevorzugt als Energiequelle einen oder mehrere UV- Strahler als Energiequelle in dem zylindrischen Stützkörper auf und der Stützkörper besteht bevorzugt aus einem für UV-Licht durchlässigen Material, z.B. Quarzglas, und weist ggf einen Wellschlauch aus Kunststoff auf.

Bevorzugt ist weiter eine Vorrichtung, dadurch gekennzeichnet, dass in die Verteilerköpfe (9, 10) Fenster (64) zur Beobachtung der in das Produkt eingestrahlten UV-Energie eingesetzt sind, die insbesondere über eine O-Ringverbindung (31, 64) in den Verteilerköpfen (9, 10) abgedichtet sind.

In einer besonders bevorzugten Ausführung sind in den Verteilerköpfen UV-Sensoren zur Erfassung der in den Produktraum eingestrahlten UV-Strahlungsintensität eingebaut.

Der Wärmebehandlungsreaktor weist besonders bevorzugt ein für Wärme durchlässiges Rohrmaterial, z.B. Edelstahl, wie 316L V4A, Chrom-Nickelstahle oder austenitischer Stahl, für das Stützrohr und einen Wellschlauch aus Kunststoff auf. Unter Kunststoff ist im Kontext dieser Erfindung bevorzugt Polytetrafluorethylen (PTFE), Perfluoroalkoxypolymere (PFA), FEP (Copolymere aus Hexafluoropropylen und Tetrafluoroethylen), PVDF (Polyvinylidenfluoride), ECTFE sowie Polypropylene und Polyethylene zu verstehen.

Zur Vergrößerung des Wärmetransportes der durch den Reaktor hindurchströmenden Temperiermedien ist bevorzugt im Zentrum des Stützrohres des Wärmebehandlungsreaktors ein Einbauelement zur Querschnittsverengung eingebaut und das Stützrohr ist von einem Temperiermedium durchströmbar.

Das querschnittverengende Einbauelement kann auch bevorzugt mit endständigen Flanschverbindungen mittels Gewinde oder vorzugsweise Bajonettverschluss als Zuganker lösbar miteinander verbunden sein, die eine Abdichtung des Innenraums des Stützrohrs bewirken.

Besonders bevorzugt ist eine Ausführung, die dadurch gekennzeichnet, dass das Einbauelement einen Radialverteiler für das Wärmeträgermedium aufweist.

Vorrichtung nach einem der Besonders bevorzugt weist das Einbauelement eine wendelförmige Innenkontur auf

Eine weitere bevorzugte Ausführung der Vorrichtung ist dadurch gekennzeichnet, dass das Stützrohr (62) auf einer Seite verschlossen ist und auf der gegenüberliegenden offenen Stützrohrseite ein Einbauelement mit Zu- und Ableitung für das Wärmeträgermedium aufweist.

Besonders bevorzugt besteht das Einbauelement aus einem angeflanschten Rohr, bei dem die Wärmeträgerzuleitung mit dem Rohrinneren verbunden ist und die Ableitung über den Spalt zwischen Einbauelement und Stützrohr erfolgt.

In einer bevorzugten Variante ist in dem Wärmebehandlungsreaktor eine elektrische, Widerstandsheizquelle angebracht, die in das Stützrohr (63) eingebaut ist.

Zur Verbesserung der Wärmeleitfähigkeit des Ringspaltes zwischen Heizquelle und Stützrohr ist der Ringspalt in einer bevorzugten Form mit einem Wärmeübertragungsmedium befüllt.

Eine weitere bevorzugte Variante der Vorrichtung ist dadurch gekennzeichnet, dass die beim Einführen oder Betreiben der Heizquelle in das Stützrohr verdrängte Wärmeübertragungsflüssigkeit in einem mit dem Stützrohr verbundenen Vorlagegefäß auffangbar ist.

Besonders bevorzugt weist der Wärmebehandlungsreaktor im Bereich seines Eingangs und Ausgangs Temperatursensoren, z.B. PT100-Platinwiderstandssensoren, auf zur Bestimmung der Wärmeträgertemperatur und /oder der Produkttemperatur.

Besonders bevorzugt ist noch eine Ausführung der Vorrichtung die dadurch gekennzeichnet ist, dass die Sensoren mit Durchflussreglern für den Wärmeträgerstrom und/oder den Produktstrom regelungstechnisch verbunden sind.

Weiterer Gegenstand der Erfindung ist ein kontinuierliches Verfahren zur Sterilisation und gegebenenfalls Virusinaktivierung fluider, insbesondere wässriger Reaktionsmedien mittels einer kombinierten Anwendung einer Wärme- und einer UV-Behandlung durch Bestrahlung in einer erfindungsgemäßen Vorrichtung, dadurch gekennzeichnet, dass die Wärmebehandlung des Reaktionsmediums bei einer Sterilisationstemperatur von 40°C bis 135°C und die Bestrahlung bei einer Bestrahlungsdichte von 5 bis 300 W/m² erfolgt.

Bevorzugt wird das Reaktionsmedium bis zu 50 sec auf der Sterilisationstemperatur gehalten.

Die Aufheizung des Reaktionsmediums auf Sterilisationstemperatur und die Abkühlung des Reaktionsmediums erfolgt bevorzugt unabhängig von einander innerhalb von 0,1 bis 10 sec.

Besonders bevorzugt ist ein Verfahren, dadurch gekennzeichnet, dass die thermische Behandlung in hintereinandergeschalteten Heiz-, Temperaturhalte- und Kühlschritten (3) stattfindet und die UV-Behandlung insbesondere während der thermischen Behandlung erfolgt.

Bevorzugt werden zur thermischen Behandlung Hochleistungswärmeaustauscher eingesetzt, die mit einem k-Wert von k > 1000 W/m²*K eine Aufheizung und Abkühlung des Produktstroms in einer Zeit von 0,1 bis 10 sec ermöglichen.

Bevorzugt ist ferner ein , bei dem für alle oder einzelne Behandlungsschritte GMP (Good Manufacturing Practice)-gereinigte, vorsterilisierte Einweg-Reaktoren eingesetzt werden.

### Beispiele:

Als Vorrichtung zur Durchführung der Sterilisation und Virusinaktivierung werden, wie in den Fig. 3, 3a und 3b dargestellt, erfindungsgemäß Reaktoren mit wendelförmigen Kanälen (8) eingesetzt. Die Herstellung der Kanäle erfolgt durch Aufziehen eines gewendelten Schlauches (5) auf einen zylindrischen Stützkörper (6). Durch eine geeignete Geometrie des gegenüber dem Stützkörper (6) im Innendurchmesser geringfügig verminderten Wellschlauches (5) wird eine straffe, kraftschlüssige Verbindung zwischen beiden Reaktor-Elementen hergestellt. Hierdurch lassen sich die sonst durch Spalte zwischen den Strömungskanälen verursachten axialen Kurzschlussströmungen verhindern, die, wie Untersuchungen belegen, eine starke Verbreiterung der Verweilzeitverteilung zur Folge hätten. Der Produktfluss ist günstigerweise aufwärts gerichtet, um einer Rückvermischung des Produktstroms durch entgegenströmende Gasblasen zu vermeiden. Ein Aufblähen des Wellschlauches (5) infolge des bei größeren Produktströmen ansteigenden Druckverlustes ist wegen der Ausbildung von Kurzschlussströmungen unerwünscht und wird erfindungsgemäß durch eine entsprechend dimensionierte Wandstärke des Wellschlauches (5) und/oder in den Wellschlauch eingelegte Metallarmierungen und/oder eine Ummantelung (21) verhindert. Die Gestaltung eines Mantelrohres ist dabei günstigerweise so festzulegen, dass der Innendurchmesser des Mantels geringfügig kleiner ist als der Schlauchaußendurchmesser, um ohne nennenswerte Schlauchdeformation einen zusätzlichen Anpressdruck zu erzeugen. Bei kleinen Druckverlusten kann ein auf den Wellschlauch einfach aufzubringender Schrumpfschlauch die Druckstabilität verbessern. Weitere ebenfalls auch nach der Modulherstellung anwendbare Mantelkonstruktionen könnten z.B. aus Halbschalen und mehrlagig gewickelten GFK-Schichten gefertigt werden.

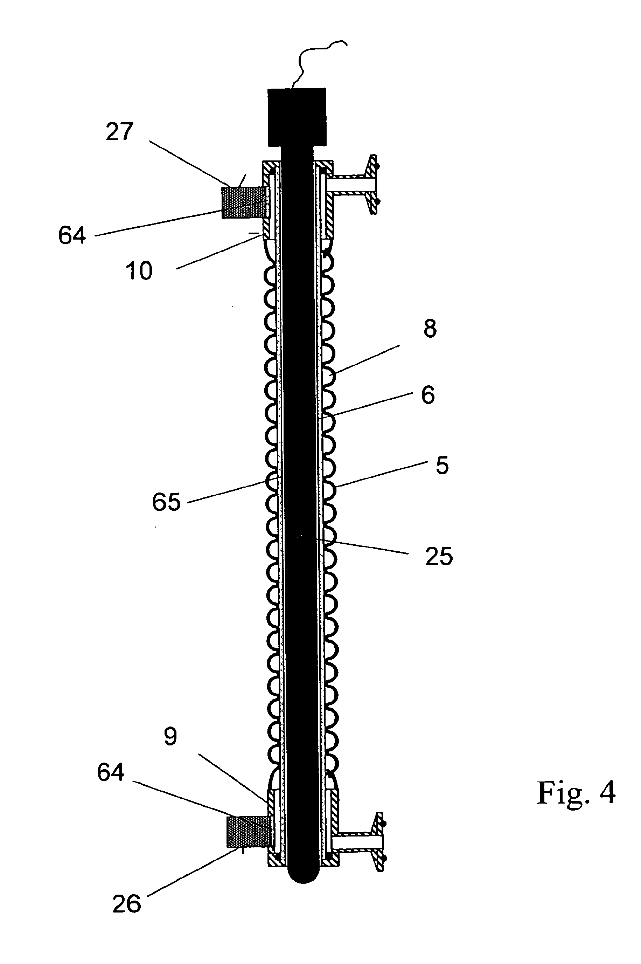

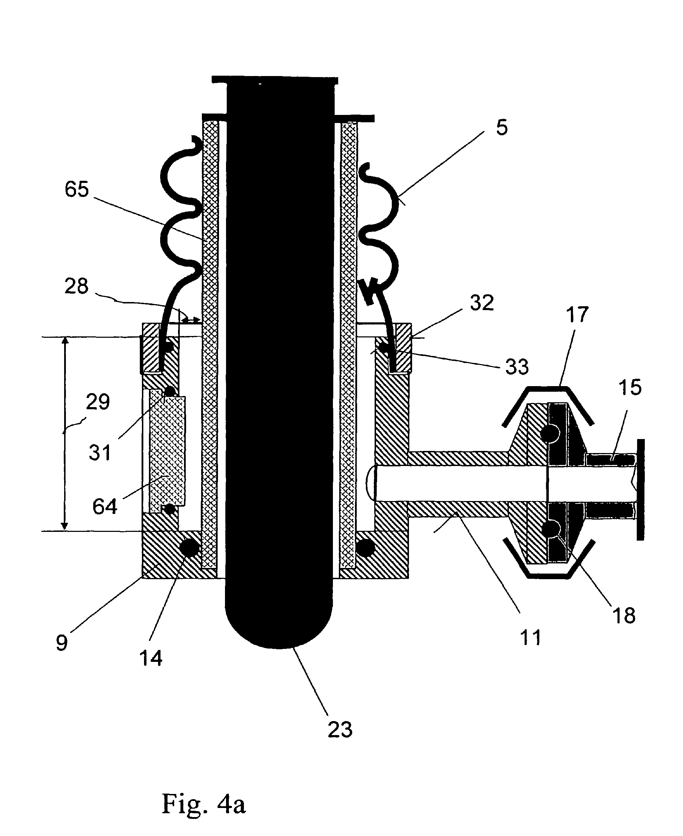

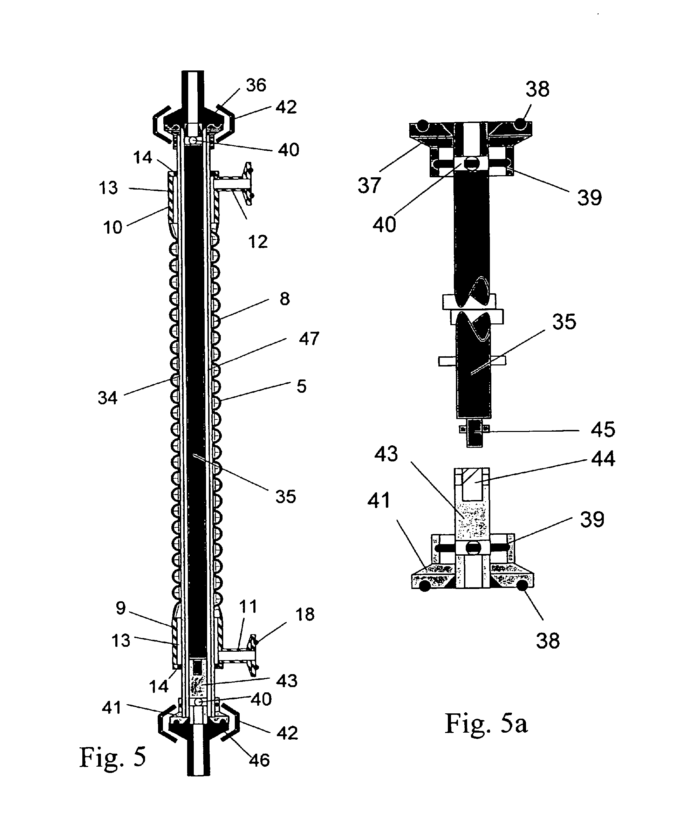

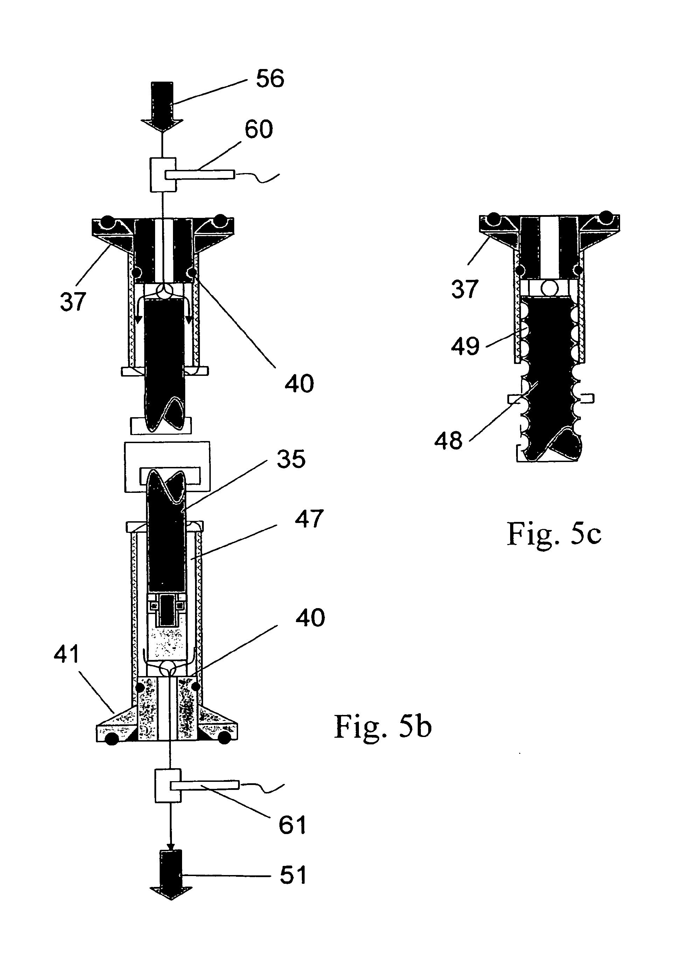

Der Energieeintrag erfolgt durch das Stützrohr (6), das zur UV-Behandlung (s. Fig. 4 und 4a) als für UV-Strahlen durchlässiges Quarzrohr (65) und zur thermischen Behandlung (s. Fig. 5, 5a, 5b und 5c) als dünnwandiges, gut wärmeleitendes Metallrohr (34) ausgeführt ist. Bei beiden Sterilisationsmethoden bilden sich Beläge auf den für den Energieeintrag genutzten Oberflächen des Stützkörpers. Eine Abreinigung dieses als Fouling bezeichneten Bewuchses ist dort möglich, wo Verschmutzungen mit dem Reinigungsmittel in Kontakt gebracht werden können. Als besonders schwer zu reinigen erweisen sich in diesem Zusammenhang die schlecht anströmbaren, für das Fouling besonders anfälligen Gebiete (22) um Kontaktstellen zwischen Schlauch und Rohr (s. Fig. 3b). Die in diesem Fall zur Durchführung einer sachgerechten GMP-Reinigung erforderliche komplette Demontage des Reaktors kann wegen des großen Zeit- und Präzisionsbedarfs von den Prozessbetreibern vor Ort nicht geleistet werden. Die Reaktoren werden deshalb erfindungsgemäß als steril verpackt angelieferte, zertifizierte, schnell und einfach austauschbare Einwegmodule in GMP-Sterilsiationsprozessen zur Verwendung empfohlen.

Der Einbau der Reaktoren erfolgt nach Öffnen der Sterilverpackung unmittelbar vor Prozessbeginn, in dem die an den identisch aufgebauten Verteilerköpfen (9, 10) vorhandenen Stutzten (11, 12) mit den Stutzen (15, 16) der Produktleitung verbunden werden. Besonders geeignet für einen schnellen, hygienischen Anschluss sind sog. Triclamp- Verbindungen bestehend aus den entsprechend geformten Flanschenden der Stutzen (11, 12, 15, 16), einer Verbindungsschelle (17) und einer Spezialdichtung (18).

Die Verteilerköpfe (9) und (10) sind spiegelsymmetrisch mit den zylindrisch aufgeweiteten Enden des Wellschlauches verbundenen. Eine hygienisch einwandfreie Verbindung wird bevorzugt durch eine O-Ringabdichtung (33, s. Fig. 4a) gewährleistet. Bei der in Fig. 4a vorgeschlagenen Abdichtung wird die Verbindung zwischen Schlauch (5) und O-Ring (33) durch Pressen von außen mittels des Ringes (32) erreicht. Weitere Verbindungsvarianten wären das Verschweißen der Verteilerköpfe mit dem Wellschlauch sowie die Integration der Verteilerköpfe in die durch entsprechende z.B. thermische Formgebung veränderten Schlauchenden des Wellschlauches. Die Abdichtung der Verteilerköpfe (9, 10) gegen das Stützrohr (6, 65, 34) erfolgt mittels O-Ringen (14).

Den Verteilerköpfen (9, 10) kommt neben der Reaktorhalterung insbesondere die Aufgabe der Vorverteilung des Produktstroms zu. Durch die spezielle Konstruktion der Verteilerköpfe wird gewährleistet, dass negative Auswirkung der Vorverteilung auf die Verweilzeitcharakteristik vermieden werden. Dies wird erfindungsgemäß durch eine starke Begrenzung des produktberührten Kopfvolumens und dieses wiederum durch eine Minimierung von Spaltweite (28) und Bauhöhe (29) erreicht. Wie Verweilzeituntersuchungen zeigen, kann bei den volumenminimierten Verteilerköpfen im allgemeinen auf eine tangentiale Zugabe und Abfuhr des Produktstromes zugunsten einer wegen der einfacheren, preiswerteren Fertigung zu bevorzugenden radialen Zugabe verzichtet werden.

Fig. 4 und 4a zeigen den für die UV-Bestrahlung vorgesehenen Reaktor. Das Stützrohr (65) des Wellschlauches ist aus Quarzglas hergestellt. In das Zentrum des Stützrohres (65) werden zur UV-Behandlung eine oder mehrere UV-Strahler (25) installiert. Zur Überwachung des Fouling-Bewuchses sind die Verteilerköpfe (9) und (10) mit Quarzfenstern (64) ausgestattet, durch die mittels der UV-Sensoren (26, 27) eine Messung des in den Kopfraum emittierten UV-Lichtes ermöglicht wird. Die von den Sensoren zur Verfügung gestellten Informationen werden erfindungsgemäß zur GMP-gerechten Dokumentation des Bestrahlungs-prozesses sowie zur Konstanthaltung der Bestrahlungsdosis durch entsprechende Anpassung der Produktverweilzeit über den Produktdurchsatz genutzt. Auf diese Weise lassen sich die Filmbildung auf dem Quarzglas und ein Verlust an Strahlungsleistung der UV-Strahlenquelle ohne Auswirkung auf die Bestrahlungsprozess kompensieren.

Fig. 5, 5a , 5b , 5c, 6 und 7 zeigen die Reaktoren zur Sterilisation durch thermischen Behandlung, die sich gleichermaßen für die Aufheizung und die Abkühlung des Produktstromes einsetzen lassen. Das Stützrohr (34) ist aus einem druckstabilen, möglichst dünnwandigen und gut leitfähigen und von der FDA zugelassenen Material hergestellt. Günstige Wärmeübertragungsverhältnisse bieten z.B. Edelstahlrohre. Durch eine Elektropolitur auf der der Produktseite zugewandten Rohrfläche kann die Bereitschaft zur Ausbildung von Fouling-Schichten auf den Heizflächen verringert werden.

Zur Hitzesterilisation werden die Module über Flanschverbindungen (36, 37, 42) und (46, 41, 42) an Heizmedien, wie z.B. Dampf oder Heißwasser, oder zur Kühlung an Kühlmedien, wie z.B. Kaltwasser oder Sohle angeschlossen. Während die Temperierflüssigkeiten die Reaktoren günstigerweise aufwärtsgerichtet durchströmen, um die Bildung von Gaseinschlüssen zu vermeiden, ist bei der Dampftemperierung zur Kondensatabfuhr eine abwärtsgerichtete Durchströmung zu bevorzugen. Um dem Verbleib von Kondensat am Boden zu verhindern können hier bevorzugt auch ein Kondensatabscheider oder ein Antibeschlagssystem angebracht sein. Insbesondere bei der Temperierung mit Flüssigkeiten ist es zur Verbesserung des heiz- bzw. kühlseitigen Wärmeüberganges im allgemeinen erforderlich, die Überströmungsgeschwindigkeit der Wärmeaustauschfläche (34) durch querschnittsverengende Einbauten (35) zu vergrößern.

Wie Fig. 5, 5 a und 5 b zeigen, kann ein solcher Einbau aus einem mit den Anschlussflanschen (37, 41) verschweißten, zweigeteilten Zylinder (35, 43) gebildet werden. Die beiden Zylinderelemente (35, 43) lassen sich mittels eines Gewindes oder zur Verringerung der O-Ringbelastung während der Montage günstigerweise mittels eines Bajonettverschlusses (44, 45) miteinander kraftschlüssig verbinden. Die zentral zugeführten Temperiermedien (50, 51) werden über die radialen Bohrungen (40) in den Ringspalt (47) zwischen Einbau (35, 43) und Stützrohr (34) verteilt und auf der gegenüberliegenden Seite über die spiegelsymmetrisch angeordneten Bohrungen (40) wieder herausgeführt. Wie Fig. 5 c zeigt, kann anstelle des zylindrischen Ringspaltes durch eine entsprechende Formgebung des in diesem Fall wandnah gegen das Stützrohr verlegten Einbauelementes (48) dem Temperiermedium eine Wendelströmung (49) aufgezwungen werden, die wegen der entstehenden Sekundärströmungen bei gleichen Druckverlusten einen zusätzlichen Beitrag zur Verbesserung des Wärmeaustausches liefert.

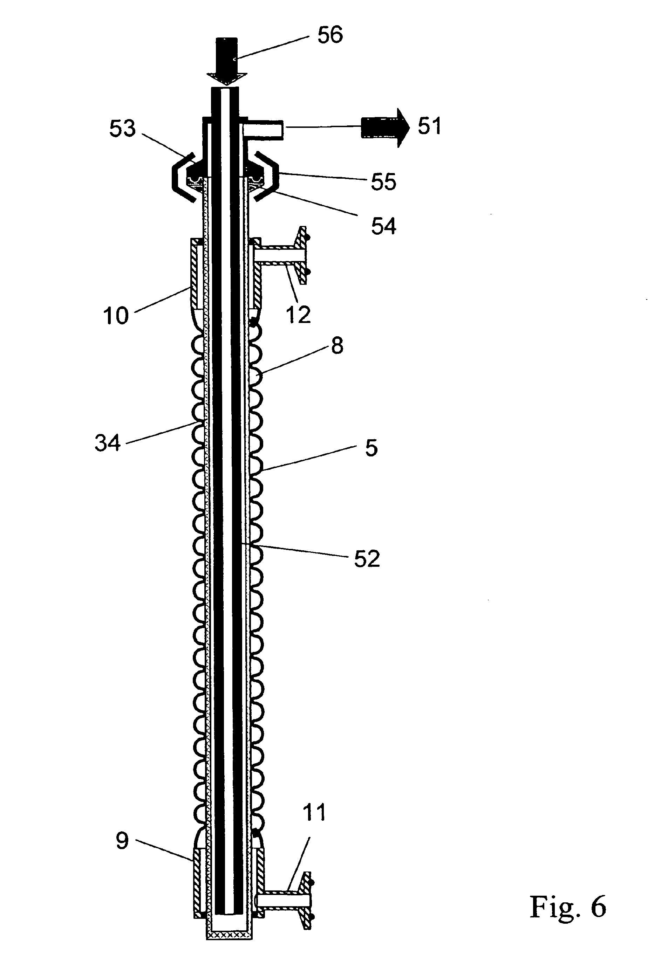

Bei dem in Fig. 6 gezeigten Reaktor. werden die flüssigen Temperiermedien durch eine Lanze (52) zur verschlossenen gegenüberliegenden Seite des Stützrohres (62) geleitet, wo diese in den gegenläufig durchströmtem Ringspalt (47) eingeleitet werden. Hierdurch wird die Handhabung der Temperiermodule erheblich vereinfacht, da durch den in das Modul integrierbaren Flansch (53) die in Fig. 5 gezeigte Vormontage der Flanschenden (37, 41) am Stützrohres (34) entfällt. Außerdem reduziert sich durch die Verlagerung des Zugabe- und Abtransportortes der Temperiermedien auf die selbe Modulseite der Montageaufwand beim Einbau in die Produktionsanlage auf die Befestigung einer einzigen Flanschverbindung (53, 54, 42). Im Falle dampfförmiger Temperiermedien wird die Strömungsrichtung zur Kondensatabfuhr umgekehrt, so dass der Dampf zunächst in den Ringspalt (47) eingeleitet wird und diesen abwärtsgerichtet durchströmt, bevor er gemeinsam mit dem Kondensat über die aufwärts durchströmte Lanze (52) abgeführt wird.

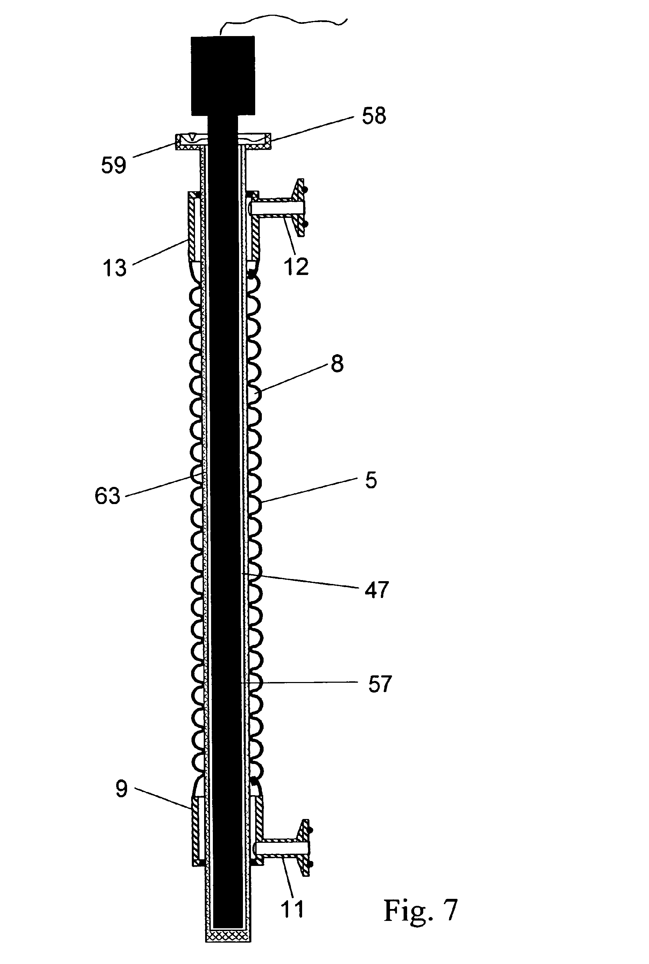

Bei der in Fig. 7 gezeigten Anordnung wird anstelle einer indirekten Beheizung über strömende Heizmedien eine direkte elektrische Beheizung über eine zylindrische Heizquelle (57) verwendet. Hierzu wird die Heizquelle (57) in das am gegenüberliegenden Ende verschlossene Stützrohr (63) eingeführt. Die auch bei dünnen luftgefüllten Ringspalten (47) zwischen Heizquelle (57) und Stützrohr (63) schlechten Wärmeleitfähigkeiten lassen sich durch Füllung mit speziellen Wärmeübertragungsmedien (59) anheben. Bei der Verwendung flüssiger Wärmeübertragungsmedien im senkrecht aufgestellten Stützrohr kann die beim Einschieben der Heizquelle (57) verdrängte Flüssigkeit von dem mit dem Stützrohr verbundenen Vorlagegefäß (58) aufgefangen werden.

## Patentansprüche

1. Vorrichtung zur Sterilisation und gegebenenfalls Virusinaktivierung flüssiger Reaktiotismedien (1) bestehend wenigstens aus einem Wärmebehandlungsreaktor (2), wobei dieser gegebenenfalls durch eine Temperaturhaltestreeke (55) erweitert ist, einem UV-Bestrahlungsreaktor (4) und einem Kühlreaktor (3), **dadurch gekennzeichnet, dass** ein von einem Reaktionsmedium (1) durchströmter Sterilisations- und/oder Virusinaktivierungsraum (8) mindestens des Bestrahlungsreaktors (4) und des Wärmebehandlungsreaktors (2) durch einen verformbaren wendelförmigen profilierten Hohlzylinder (5) gebildet wird, der auf einen für eingesetzte Sterilisations- oder Virusinaktivierungsenergie (7) durchlässigen, starren, geraden, zylindrischen Stützkörper (6) kraftschlüssig aufgezogen ist.

2. Vorrichtung nach Anspruch 1, **dadurch gekennzeichnet, dass** als ein verformbarer wendelförmiger profilierter Hohlzylinder (5) ein Wellschlauch aus Kunststoff verwendet wird, der zur Produktzu- bzw. -abfuhr an beiden Enden mit Verteilerköpfen (9, 10) verbunden ist.

3. Vorrichtung nach Anspruch 2, **dadurch gekennzeichnet, dass** die Verteilerköpfe (9, 10) eine totraumfreie tangentiale oder vorzugsweise radiale Produktzu- bzw. -ableitung im Bereich des Ringspaltes (13) zwischen Verteilerkopf (9, 10) und Stützrohr (6) aufweisen.

4. Vorrichtung nach Anspruch 2 bis 3, **dadurch gekennzeichnet, dass** die Verteilerköpfe (9, 10) durch eine nachträgliche thermische Verformung des Wellschlauches aus den Schlauchenden herausgearbeitet sind oder bevorzugt aus einem im Spritzgusaverfahren und/oder spanend hergestellten bzw. bearbeiteten Kunststoffmaterial hergestellt sind und mittels einer von außen angepressten O-Ring-Verbindung (32, 5, 33) mit den zylindrisch aufgeweiteten Schlauchenden kraftschlüssig verbunden sind.

5. Vorrichtung nach einem der Ansprüche 2 bis 4, **dadurch gekennzeichnet, dass** der Wellschlauch (5) einen Außenmantel (21) oder eine Armierung (50) aufweist.

6. Vorrichtung nach Anspruch 5, **dadurch gekennzeichnet, dass** der Außenmantel (21) durch einen schrumpfbaren Kunststoffschlauch, ein über einen Spiralschlauch geschobenes Rohr oder vorzugsweise eine zweigeteilte, zylinderische Schale gebildet ist, wobei die Armierung aus einer Stahl- oder Kunststoffwendel besteht.

7. Vorrichtung nach einem der Ansprüche 1 bis 6, **dadurch gekennzeichnet, dass** der UV-Reaktor als Energiequelle (7) einen oder mehrere UV- Strahler (25) als Energiequelle in dem zylindrischen Stützkörper (6) aufweist und der Stützkörper (6) aus einem für UV-Licht durchlässigen Material, z.B. Quarzglas, besteht.

8. Vorrichtung nach Anspruch 2 bis 4, **dadurch gekennzeichnet, dass** in die Verteilerköpfe (9, 10) Fenster (64) zur Beobachtung der in das Produkt eingestrahlten UV-Energie eingesetzt sind, die insbesondere über eine O-Ringverbindung (31, 64) in den Verteilerköpfen (9, 10) abgedichtet sind.

9. Vorrichtung nach einem der Ansprüche 2 bis 4 oder 8, **dadurch gekennzeichnet, dass** in den Verteilerköpfen (9, 10) UV-Sensoren (26, 27) zur Erfassung der in den Produktraum eingestrahlten UV-Strahlungsintensität eingebaut sind.

10. Vorrichtung nach einem der Ansprüche 1 bis 9, **dadurch gekennzeichnet, dass** der Wärmebehandlungsreaktor (2) ein für Wärme durchlässiges Rohrmaterial, z.B. Edelstahl für das Stützrohr (34) und einen Wellschlauch (5) aus Kunststoff aufweist.

11. Vorrichtung nach einem der Ansprüche 1 bis 10, **dadurch gekennzeichnet, dass** zur Vergrößerung des Wärmetransportes der durch den Reaktor hindurchströmenden Temperiermedien im Zentrum des Stützrohres (34) des Wärmebehandlungsreaktors (2) ein Einbauelement (35, 43) zur Querschnittsverengung eingebaut ist und das Stützrohr (34) von einem Temperiermedium durchströmbar ist.

12. Vorrichtung nach Anspruch 11, **dadurch gekennzeichnet, dass** das querschnittverengende Einbauelement (35, 43) mit endständigen Flanschverbindungen mittels Gewinde oder vorzugsweise Bajonettverschluss (44, 45) als Zuganker lösbar miteinander verbunden ist, die eine Abdichtung des Innenraums des Stützrohrs (34) bewirken.

13. Vorrichtung nach einem der Ansprüche 11 bis 12, **dadurch gekennzeichnet, dass** das Einbauelement (35, 43) einen Radialverteiler (40) für das Wärmeträgermedium aufweist.

14. Vorrichtung nach einem der Ansprüche 11 bis 13, **dadurch gekennzeichnet, dass** das Einbauelement (35, 43) eine wendelförmige Innenkontur aufweist.

15. Vorrichtung nach einem der Ansprüche 1 bis 14, **dadurch gekennzeichnet, dass** das Stützrohr (62) auf einer Seite verschlossen ist und auf der gegenüberliegenden offenen Stützrohrseite ein Einbauelement (35, 43, 52) mit Zu- und Ableitung für das Wärmeträgermedium aufweist.

16. Vorrichtung nach Anspruch 15, **dadurch gekennzeichnet, dass** das Einbauelement (35, 43, 52) aus einem angeflanschten Rohr besteht, bei dem die Wärmeträgerzuleitung mit dem Rohrinneren verbunden ist und die Ableitung über den Spalt (47) zwischen Einbauelement und Stützrohr (62) erfolgt.

17. Vorrichtung nach einem der Ansprüche 1 bis 10, **dadurch gekennzeichnet, dass** in dem Wärmebehandlungsreaktor (2) eine elektrische, Widerstandsheizquelle (57) angebracht ist, die in das Stützrohr (63) eingebaut ist.

18. Vorrichtung nach Anspruch 17, **dadurch gekennzeichnet, dass** zur Verbesserung der Wärmeleitfähigkeit des Ringspaltes (47) zwischen Heizquelle (57) und Stützrohr (63) der Ringspalt mit einem Wärmeübertragungsmedium (59) befüllt ist.

19. Vorrichtung nach einem der Ansprüche 17 bis 18, **dadurch gekennzeichnet, dass** die beim Einführen oder Betreiben der Heizquelle (57) in das Stützrohr (62) verdrängte Wärmeübertragungsflüssigkeit (59) in einem mit dem Stützrohr verbundenen Vorlagegefäß (58) au ffan gbar ist.

20. Vorrichtung nach einem der Ansprüche 1 bis 19, **dadurch gekennzeichnet, dass** der Wärmebehandlungsreaktor (2) im Bereich seines Eingangs und Ausgangs Temperatursensoren, z.B. PT100-Widerstandssensoren, aufweist zur Bestimmung der Wärmeträgertemperatur und /oder der Produkttemperatur.

21. Vorrichtung nach einem der Ansprüche 9 bis 20, **dadurch gekennzeichnet, dass** die Sensoren (60, 61) mit Durchflussreglern für den Wärmeträgerstrom und/oder den Produktstrom regelungstechnisch verbunden sind.

22. Kontinuierliches Verfahren zur Sterilisation und gegebenenfalls Virusinaktivierung flüssiger, insbesondere wässriger Reaktionsmedien (1) mittels einer kombinierten Anwendung einer Wärme- (2, 3, 55) und einer UV-Behandlung durch Bestrahlung (4) in einer Vorrichtung gemäß einem der Ansprüche 1 bis 21, **dadurch gekennzeichnet, dass** die Wärmebehandlung des Reaktionsmediums bei einer Sterilisationstemperatur von 40°C bis 135°C und die Bestrahlung bei einer Bestrahlungsdichte von 5 bis 300 W/m² erfolgt.

23. Verfahren nach Anspruch 22, **dadurch gekennzeichnet, dass** das Reaktionsmedium bis zu 50 sec auf der Sterilisationstemperatur gehalten wird.

24. Verfahren nach Anspruch 22 oder 23, **dadurch gekennzeichnet, dass** die Aufheizung des Reaktionsmediums auf Sterilisationstemperatur und die Abkühlung des Reaktionsmediums unabhängig von einander innerhalb von 0,1 bis 10 sec erfolgt.

25. Verfahren nach einem der Ansprüche 22 bis 24, **dadurch gekennzeichnet, dass** die thermische Behandlung in hintereinandergeschalteten Heiz- (2), Temperaturhalte- (55) und Kühlschritten (3) stattfindet und die UV-Behandlung (4) insbesondere während der thermischen Behandlung erfolgt.

26. Verfahren nach einem der Ansprüche 22 bis 25, **dadurch gekennzeichnet, dass** zur thermischen Behandlung Hochleistungswärmeaustauscher eingesetzt werden, die mit einem k-Wert von k > 1000 W/m²*K eine Aufheizung und Abkühlung des Produktstroms in einer Zeit von 0,1 bis 10 sec ermöglichen.

27. Verfahren nach einem der Ansprüche 22 bis 25, **dadurch gekennzeichnet, dass** für alle oder einzelne Behandlungsschritte GMP-gereinigte, vorsterilisierte Einweg-Reaktoren eingesetzt werden.

## Claims

1. Apparatus for sterilization and, if appropriate, virus inactivation of liquid reaction media (1) consisting at least of a heat treatment reactor (2), said reactor if appropriate being expanded by a temperature holding section (55), a UV irradiation reactor (4) and a cooling reactor (3), **characterized in that** a sterilization and/or virus inactivation chamber (8) through which a reaction medium (1) flows, at least of the irradiation reactor (4) and of the heat treatment reactor (2), is formed by a deformable, helical, profiled hollow cylinder (5) which is drawn in a force-fitting manner onto a rigid, straight, cylindrical support body (6) transparent to the sterilization or virus inactivation energy (7) used.

2. Apparatus according to Claim 1, **characterized in that** the deformable, helical, profiled hollow cylinder (5) used is a corrugated plastic hose which, for product delivery and discharge, is connected at both ends to distributor heads (9, 10).

3. Apparatus according to Claim 2, **characterized in that** the distributor heads (9, 10) have tangential or preferably radial product delivery and discharge lines, free of dead space, in the area of the annular gap (13) between distributor head (9, 10) and support pipe (6).

4. Apparatus according to Claims 2 to 3, **characterized in that** the distributor heads (9, 10) are worked from the hose ends by subsequent thermal deformation of the corrugated hose or are preferably produced from a plastic material that has been produced or worked by injection moulding and/or cutting and are connected with a force fit to the cylindrically widened hose ends by means of an O-ring connection (32, 5, 33) pressed on from outside.

5. Apparatus according to one of Claims 2 to 4, **characterized in that** the corrugated hose (5) has an outer jacket (21) or a reinforcement (50).

6. Apparatus according to Claim 5, **characterized in that** the outer jacket (21) is formed by a shrinkable plastic tube, a pipe pushed over the spiral hose, or preferably a two-part, cylindrical shell, the reinforcement being formed by a steel or plastic coil.

7. Apparatus according to one of Claims 1 to 6, **characterized in that** the UV reactor has, as energy source (7), one or more UV emitters (25) as energy source in the cylindrical support body (6), and the support body (6) is made of a material transparent to UV light, e.g. quartz glass.

8. Apparatus according to Claims 2 to 4, **characterized in that** the distributor heads (9, 10) have windows (64) for observing the UV energy radiated into the product, which windows (64) are in particular sealed off by an O-ring connection (31, 64) in the distributor heads (9, 10).

9. Apparatus according to one of Claims 2 to 4 or 8, **characterized in that** UV sensors (26, 27) are built into the distributor heads (9, 10) for the purpose of detecting the UV radiation intensity radiated into the product chamber.

10. Apparatus according to one of Claims 1 to 9, **characterized in that** the heat treatment reactor (2) has a pipe material transmitting heat, for example stainless steel for the support pipe (34), and a corrugated hose (5) made of plastic.

11. Apparatus according to one of Claims 1 to 10, **characterized in that**, in order to increase the heat transfer of the temperature control media flowing through the reactor, an insert element (35, 43) is incorporated in the centre of the support pipe (34) of the heat treatment reactor (2) so as to narrow the cross section, and a temperature control medium can flow through the support pipe (34).

12. Apparatus according to Claim 11, **characterized in that** the insert element (35, 43) narrowing the cross section has terminal flange connections which are connected releasably by means of a thread or preferably a bayonet closure (44, 45) and seal off the inner space of the support pipe (34).

13. Apparatus according to one of Claims 11 to 12, **characterized in that** the insert element (35, 43) has a radial distributor (40) for the heat transfer medium.

14. Apparatus according to one of Claims 11 to 13, **characterized in that** the insert element (35, 43) has a helical inner contour.

15. Apparatus according to one of Claims 1 to 14, **characterized in that** the support pipe (62) is closed at one end and has, at the other, open end of the support pipe, an insert element (35, 43, 52) with inlet and outlet line for the heat transfer medium.

16. Apparatus according to Claim 15, **characterized in that** the insert element (35, 43, 52) consists of a flanged pipe in which the heat transfer medium inlet line is connected to the pipe interior and the outlet is via the gap (47) between insert element and support pipe (62).

17. Apparatus according to one of Claims 1 to 10, **characterized in that** an electrical resistance heating source (57), fitted into the support pipe (63), is arranged in the heat treatment reactor (2).

18. Apparatus according to Claim 17, **characterized in that**, in order to improve the heat conductivity of the annular gap (47) between heating source (57) and support pipe (63), the annular gap is filled with a heat transfer medium (59).

19. Apparatus according to one of Claims 17 to 18, **characterized in that** the heat transfer fluid (59) displaced into the support pipe (62) upon insertion or operation of the heat source (57) can be collected in a receiving vessel (58) connected to the support pipe.

20. Apparatus according to one of Claims 1 to 19, **characterized in that**, in the area of its inlet and outlet, the heat treatment reactor (2) has temperature sensors, e.g. PT100 resistance sensors, for determining the heat transfer medium temperature and/or the product temperature.

21. Apparatus according to one of Claims 9 to 20, **characterized in that** the sensors (60, 61) are connected to flow regulators for the heat transfer medium stream and/or the product stream.

22. Continuous process for sterilization and, if appropriate, virus inactivation of liquid media, in particular of aqueous reaction media (1), by means of a combined application of a heat treatment (2, 3, 55) and a UV irradiation treatment (4) in an apparatus according to one of Claims 1 to 21, **characterized in that** the heat treatment of the reaction medium takes place at a sterilization temperature of 40°C to 135°C and the irradiation takes place at an irradiation density of 5 to 300 W/m².

23. Process according to Claim 22, **characterized in that** the reaction medium is held at the sterilization temperature for up to 50 seconds.

24. Process according to Claim 22 or 23, **characterized in that** the heating of the reaction medium to the sterilization temperature and the cooling of the reaction medium take place independently of one another within 0.1 to 10 seconds.

25. Process according to one of Claims 22 to 24, **characterized in that** the thermal treatment takes place in successive steps of heating (2), temperature holding (55), and cooling (3), and the UV treatment (4) takes place in particular during the thermal treatment.

26. Process according to one of Claims 22 to 25, **characterized in that** the thermal treatment is carried out using high-performance heat exchangers which, with a k value of k > 1000 W/m²*K, permit a heating and cooling of the product stream in a time of 0.1 to 10 seconds.

27. Process according to one of Claims 22 to 25, **characterized in that** all or some of the treatment steps are carried out using pre-sterilized disposable reactors cleaned according to GMP.

## Revendications

1. Dispositif pour la stérilisation et éventuellement l'inactivation virale de milieux réactionnels liquides (1) consistant au moins en un réacteur de traitement thermique (2), où celui-ci est éventuellement augmenté d'une section de maintien en température (55), un réacteur d'irradiation UV (4) et un réacteur de refroidissement (3), **caractérisé en ce qu'**une cavité de stérilisation et/ou d'inactivation virale (8) traversée par un milieu réactionnel (1) d'au moins le réacteur d'irradiation (4) et le réacteur de traitement thermique (2) est formée par un cylindre creux (5) profilé en forme d'hélice déformable qui est appliqué par assemblage de force sur un corps de support (6) cylindrique rectiligne rigide perméable à l'énergie de stérilisation ou d'inactivation virale (7) utilisée.

2. Dispositif selon la revendication 1, **caractérisé en ce qu'**un tuyau ondulé en matière synthétique est utilisé comme cylindre creux (5) profilé en forme d'hélice déformable, qui est relié aux deux extrémités à des têtes de répartition (9, 10) pour l'introduction et l'évacuation des produits.

3. Dispositif selon la revendication 2, **caractérisé en ce que** les têtes de répartition (9, 10) comportent un conduit d'apport de produit et un conduit d'évacuation de produit tangentiel ou de préférence radial sans espace mort dans le domaine de l'interstice annulaire (13) entre la tête de répartition (9, 10) et le tube de support (6).

4. Dispositif selon les revendications 2 à 3, **caractérisé en ce que** les têtes de répartition (9, 10) sont formées par une déformation thermique ultérieure du tuyau ondulé à partir des extrémités du tuyau ou de préférence sont produites à partir d'un matériau de type matière synthétique produit ou transformé dans le procédé de moulage par injection et/ou par tension et sont reliées par assemblage de force avec des extrémités élargies cylindriquement du tuyau au moyen d'une liaison par joint torique (32, 5, 33) appliquée par pression depuis l'extérieur,

5. Dispositif selon l'une des revendications 2 à 4, **caractérisé en ce que** le tuyau ondulé (5) comporte une enveloppe externe (21) ou une armature (50).

6. Dispositif selon la revendication 5, **caractérisé en ce que** l'enveloppe externe (21) est formée par un tuyau en matière synthétique rétractable, un tube enfilé sur un tuyau en spirale ou de préférence une coquille cylindrique en deux parties, où l'armature consiste en une spirale en acier ou en matière synthétique.

7. Dispositif selon l'une des revendications 1 à 6, **caractérisé en ce que** le réacteur UV comporte comme source d'énergie (7) un ou plusieurs émetteurs de rayonnement UV (25) comme source d'énergie dans le corps de support cylindrique (6) et le corps de support (6) consiste en un matériau perméable à la lumière UV, par exemple en verre de quartz.

8. Dispositif selon les revendications 2 à 4, **caractérisé en ce que** des fenêtres (64) sont prévues dans les têtes de répartition (9, 10) pour l'observation de l'énergie UV projetée dans le produit, qui sont étanchéifiées en particulier par le biais d'une liaison par joint torique (31, 64) dans les têtes de répartition (9, 10).

9. Dispositif selon l'une des revendications 2 à 4 ou 8, **caractérisé en ce que** des capteurs UV (26, 27) pour mesurer l'intensité du rayonnement UV projeté dans la cavité de produit sont incorporés dans les têtes de répartition (9, 10).

10. Dispositif selon l'une des revendications 1 à 9, **caractérisé en ce que** le réacteur de traitement thermique (2) comporte un matériau de tube perméable à la chaleur, par exemple l'acier spécial pour le tube de support (34) et un tuyau ondulé (5) en matière synthétique.

11. Dispositif selon l'une des revendications 1 à 10, **caractérisé en ce que**, pour augmenter le transport de chaleur des agents de maintien en température traversant le réacteur, un élément incorporé (35, 43) pour rétrécir la section transversale est incorporé au centre du tube de support (34) du réacteur de traitement thermique (2) et le tube de support (34) peut être traversé par un agent de maintien en température.

12. Dispositif selon la revendication 11, **caractérisé en ce que** l'élément incorporé rétrécissant la section transversale (35, 43) est relié de manière amovible avec des liaisons par brides terminales au moyen d'un filetage ou de préférence d'une fermeture à baïonnette (44, 45) sous forme de tirants qui réalisent une étanchéification de la cavité interne du tube de support (34).

13. Dispositif selon l'une des revendications 11 à 12, **caractérisé en ce que** l'élément incorporé (35, 43) comporte un répartiteur radial (40) pour l'agent caloporteur.

14. Dispositif selon l'une des revendications 11 à 13, **caractérisé en ce que** l'élément incorporé (35, 43) comporte un contour interne en forme d'hélice.

15. Dispositif selon l'une des revendications 1 à 14, **caractérisé en ce que** le tube de support (62) est fermé d'un côté et comporte du côté ouvert opposé du tube de support un élément incorporé (35, 43, 52) avec un conduit d'apport et un conduit d'évacuation pour l'agent caloporteur.

16. Dispositif selon la revendication 15, **caractérisé en ce que** l'élément incorporé (35, 43, 52) consiste en un tube fixé par brides dans lequel le conduit d'apport de caloporteur est relié avec l'intérieur du tube et le conduit d'évacuation est réalisé par le biais de l'interstice (47) entre l'élément incorporé et le tube de support (62).

17. Dispositif selon l'une des revendications 1 à 10, **caractérisé en ce qu'**une source de chaleur par résistance électrique (57), qui est incorporée dans le tube de support (63), est disposée dans le réacteur de traitement thermique (2).

18. Dispositif selon la revendication 17, **caractérisé en ce que**, pour améliorer la conductivité thermique de l'interstice annulaire (47) entre la source de chaleur (57) et le tube de support (63), l'interstice annulaire est rempli d'un agent de transmission de la chaleur (59).

19. Dispositif selon l'une des revendications 17 à 18, **caractérisé en ce que** le liquide de transmission de la chaleur (59) déplacé lors de l'insertion ou du fonctionnement de la source de chaleur (57) dans le tube de support (62) peut être capté dans un récipient collecteur (58) relié au tube de support.

20. Dispositif selon l'une des revendications 1 à 19, **caractérisé en ce que** le réacteur de traitement thermique (2) comporte dans le domaine de son entrée et de sa sortie des capteurs de température, par exemple des capteurs résistifs PT100, pour la détermination de la température du caloporteur et/ou de la température du produit.

21. Dispositif selon l'une des revendications 9 à 20, **caractérisé en ce que** les capteurs (60, 61) sont reliés à des régulateurs de débit pour le courant de caloporteur et/ou le courant de produit par la technologie de régulation.

22. Procédé continu pour la stérilisation et éventuellement l'inactivation virale de milieux réactionnels liquides, en particulier aqueux (1), au moyen d'une utilisation combinée d'un traitement thermique (2, 3, 55) et d'un traitement UV par irradiation (4) dans un dispositif selon l'une des revendications 1 à 21, **caractérisé en ce que** le traitement thermique du milieu réactionnel a lieu à une température de stérilisation de 40°C à 135°C et l'irradiation a lieu à une densité d'irradiation de 5 à 300 W/m².

23. Procédé selon la revendication 22, **caractérisé en ce que** le milieu réactionnel est maintenu jusqu'à 50 s à la température de stérilisation.

24. Procédé selon la revendication 22 ou 23, **caractérisé en ce que** le chauffage du milieu réactionnel à la température de stérilisation et le refroidissement du milieu réactionnel ont lieu indépendamment l'un de l'autre en 0,1 à 10 s.

25. Procédé selon l'une des revendications 22 à 24, **caractérisé en ce que** le traitement thermique a lieu dans des étapes de chauffage (2), de maintien en température (55) et de refroidissement (3) disposées les unes derrière les autres et le traitement UV (4) a lieu en particulier pendant le traitement thermique.

26. Procédé selon l'une des revendications 22 à 25, **caractérisé en ce que** des échangeurs de chaleur à haut rendement qui, avec une valeur k de k > 1000 W/m²*K, permettent un chauffage et un refroidissement du courant de produit en une durée de 0,1 à 10 s, sont utilisés pour le traitement thermique.

27. Procédé selon l'une des revendications 22 à 25, **caractérisé en ce que** des réacteurs unidirectionnels pré-stérilisés nettoyés par GMP sont-utilisés pour toutes les étapes de traitement ou pour des étapes de traitement individuelles.
